# EUROPEAN PATENT SPECIFICATION

(11) **EP 0 953 363 B1**
(45) Date of publication and mention of the grant of the patent: **29.12.2004**
(21) Application number: 99303181.4
(22) Date of filing: 23.04.1999
(51) Int. Cl.: A61M 25/00

(54) **Manufacture method of medico-surgical tubes**
Herstellungsmethode der medizinisch-chirurgischen Schläuche
Méthodes de fabrication des tuyaux médico-chirurgicaux

(30) Priority: 01.05.1998 GB 9809246
(43) Date of publication of application: 03.11.1999
(73) Proprietor: Smiths Group plc, London, NW11 8DS (GB)
(72) Inventor: Field, Stephen James, Bridge, Canterbury CT4 5LA (GB); Batchelor, Kester Julian, Burnham-on-Sea, Somerset TA8 2RY (GB)
(74) Representative: Flint, Jonathan McNeill

(56) References cited:
- EP-A- 0 827 758
- US-A- 4 636 346
- US-A- 4 753 765
- US-A- 5 348 536
- US-A- 5 558 737

## Description

This invention relates to methods of making medico-surgical tubes.

The invention is more particularly concerned with the manufacture of tubes with a soft tip.

It is often desirable for medico-surgical tubes, or catheters, to have a soft tip, so as to reduce trauma caused when the tip contacts patient tissue. In epidural catheters, a soft tip reduces the risk that the catheter will damage the dura. Various arrangements have been proposed for providing a soft tip, such as by attaching or moulding onto the shaft of the catheter a separate component of a softer material. Such an arrangement is not entirely satisfactory because a separate assembly operation is needed to form the tip, leading to increased manufacturing expense. Also, there is always some risk that a separate component might become detached from the body of the catheter. Other arrangements in which the rear part of the catheter is reinforced can also be difficult to make by automated assembly, thereby making the catheter relatively expensive. GB9906349 and US 4 753 765 describe a catheter with a soft tip formed by extruding a tube with an inner layer of stiffer material, which is periodically interrupted to provide more flexible regions. In WO 94/01160 there is described an epidural catheter where an inner tube is preformed and then an outer tube is provided around it extending beyond the inner tube to form a less stiff region.

It is an object of the present invention to provide an alternative method of manufacture of a such a tube.

According to the present invention there is provided a method of making a medico-surgical tube, characterised in that the method includes the steps of forming a tubular member with an inner layer and an outer layer, and subsequently removing a part at least of the inner layer along a region at one end of the tube such as to reduce the stiffness of the tube along the region.

The part of the inner layer may be removed by machining away the part of the inner layer along the region. The part of the inner layer may be machined away using a machine having a rotating milling head with a milling surface on an end face. Alternatively, the part of the inner layer may be removed by cutting away the part from the remainder of the inner layer and pulling it out of the tube. The part of the inner layer may be cut away using a machine having a rotating spindle carrying a radially-extending knife blade. Preferably the tube has an interlayer, such as of polyethylene, between the inner and outer layers preventing bonding between the inner and outer layers. The outer layer is preferably end formed after removal of the part of the inner layer to close the one end of the tube, a side opening being formed in the tube towards the one end.. The inner layer may be of a stiffer material than the outer layer.

An epidural catheter and a method of making an epidural catheter according to the present invention, will now be described, by way of example, with reference to the accompanying drawings, in which:
- Figure 1: is a side elevation view of the catheter;
- Figure 2: is an enlarged cross-sectional side elevation view of the patient end of the catheter;
- Figures 3 and 4: are enlarged cross-sectional side elevation views showing stages in a method of manufacture of the catheter; and
- Figures 5 and 6: are an enlarged cross-sectional side elevation views showing stages in an alternative method of manufacture of the catheter.

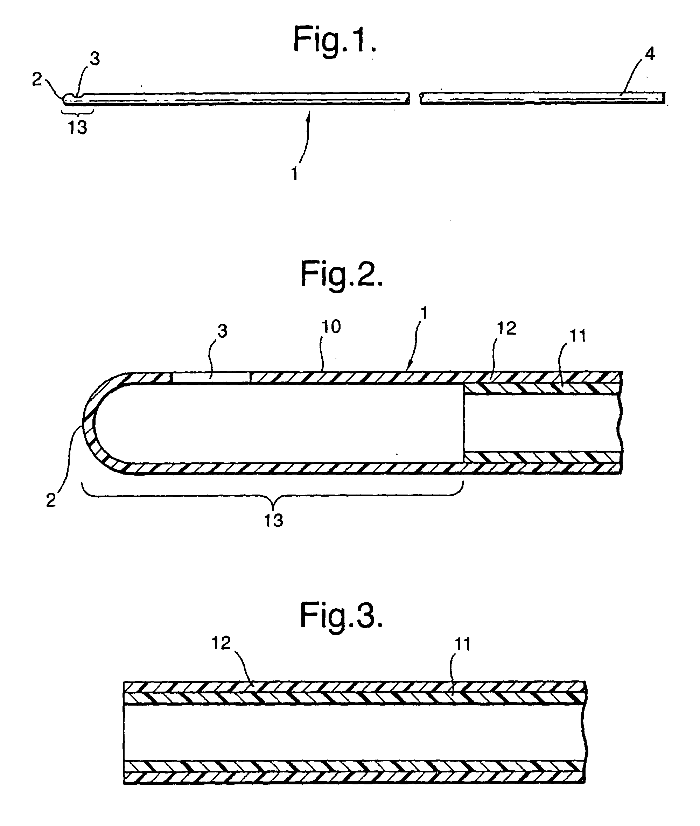

With reference first to Figure 1, the catheter 1 is about 75-100cm long with a rounded tip at its patient end 2 and a side opening 3. The machine end 4 of the catheter 1 is open and cut square for attachment to a conventional epidural connector, not shown.

Referring now also to Figure 2, the wall 10 of the catheter 1 is extruded from a thermoplastics material. The wall 10 has an inner layer 11 of nylon and an outer layer 12 of polyurethane, the nylon being stiffer than the polyurethane. Alternatively, both layers 11 and 12 may be of the same polymer, such as PVC, but with differing amounts of plasticizer so that the inner layer is stiffer. Towards the patient end 2 of the catheter 1, there is a region 13 about 2cm long where the inner layer 11 has been removed, thereby making this more flexible than the remainder of the catheter 1, and making the tip 2 relatively soft. This reduces the risk of damage to the dura and enables the forward end of the catheter 1 to bend more easily to conform to the shape of the epidural space with a reduced risk of kinking. Because the inner layer 11 is stiffer than the outer layer 12, it can be relatively thin and still provide sufficient rigidity to the main part of the catheter. This ensures that the internal diameter of the catheter 1 is kept as large as possible.

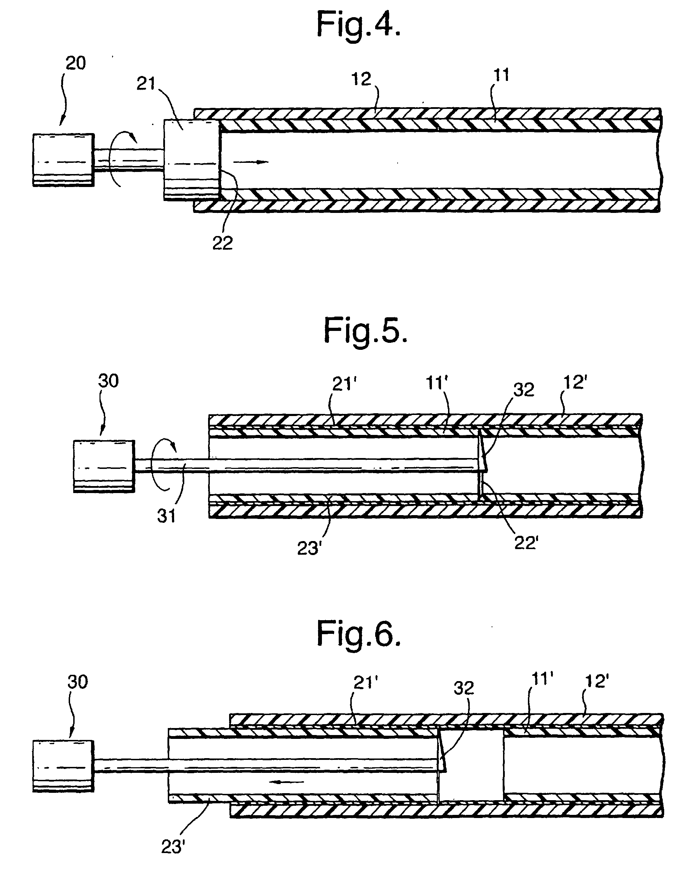

The inner layer 11 can be removed from the tip of the catheter in various ways, such as illustrated in Figures 3 and 4. Figure 3 shows tubing as it emerges from a co-extruder, having an inner layer 11 extending along its entire length. The tubing is then cut into lengths and the inner layer 11 is subsequently removed by machining it away, such as with a milling machine 20 of the kind shown in Figure 4. The machine 20 has a milling head 21 of cylindrical shape with a milling surface formed on its front face 22. The diameter of the head 21 is equal to the external diameter of the inner layer 11. The milling head 21 is rotated about its axis and is pushed axially into the tubing so as to cut away the inner layer 11 and to leave the outer layer 12. The head 21 is pushed in until the inner layer 11 has been machined away along the region 13. The machine 20 is then removed, the swarf is flushed from the tube and the tube is end formed in a conventional manner, such as by means of a heated mould, to close and round the end. The side eye 3 can be formed at any time.

It is not essential to remove the entire thickness of the inner layer 11 since some reduction in stiffness can be achieved by removing only an inner part of the thickness of the layer.

An alternative way of removing the inner layer 11' is shown in Figures 5 and 6. In this method, the tubing is extruded with an additional interlayer 21' of polyethylene between the outer layer 12' and the inner layer 11', which serves to prevent bonding between the inner and outer layers. After cutting into appropriate lengths, a cutting machine 30 is used to remove the inner layer 11'. The cutting machine 30 has a rotatable spindle 31 carrying a radially-extending knife blade 32 at one end. The blade 32 is inserted, while stationary, into the end of the tube to a depth equal to that of the region 13 from which the inner layer 11' is to be removed. The machine 30 then rotates and centres the spindle 31 within the tube. The length of the knife blade 32 is selected so that its tip traces a circle of diameter equal to the external diameter of the inner layer 11' and thereby produces a circular cut 22' through the inner layer without penetrating the outer layer 12'. The spindle 31 and blade 32 are then stopped rotating and are pulled out of the tube, as shown in Figure 6, with the blade pulling out the section 23' of the inner layer 11' forwardly of the cut 22', which has been separated from the remainder of the inner layer. The interlayer 21' ensures that this section 23' of the inner layer 11' can be slid relative to the outer layer 12'. It is not important whether the knife blade 32 cuts through the interlayer 21' and whether this is removed with the section 23' of the inner layer 11', or whether it remains within the outer layer 12'. Alternatively, a separate tool could be used to remove the section 23'. The end of the tube is then formed in the manner described above. This arrangement has the advantage that less or no swarf is produced.

The method of making the catheter 1 enables a soft patient end tip 2 to be provided without the need for subsequent assembly operations.

It will be appreciated that the invention is not confined to epidural catheters but could be used to provide a tip of reduced stiffness to other tubes such as endotracheal tubes. The catheter could be reinforced such as by incorporating a helical reinforcing element, or a braid into the outer layer. A lumen could be formed along the outer layer for various conventional purposes. The tip could be open or closed.

## Claims

1. A method of making a medico-surgical tube (1), the method including the step of forming a tubular member with an inner layer (11) and an outer layer (12), the method being **characterized by** the step of subsequently removing a part at least of the inner layer along a region (13) at one end (2) of the tube such as to reduce the stiffness of the tube along the region (13).

2. A method according to Claim 1, **characterised in that** the part of the inner layer is removed by machining away the part of the inner layer (11) along the region (13).

3. A method according to Claim 2, **characterised in that** the part of the inner layer (11) is machined away using a machine (20) having a rotating milling head (21) with a milling surface (22) on an end face.

4. A method according to Claim 1, **characterised in that** the part of the inner layer (11) is removed by cutting away the part (23') from the remainder of the inner layer and pulling it out of the tube.

5. A method according to Claim 4, **characterised in that** the part (23') of the inner layer (11) is cut away using a machine (30) having a rotating spindle (31) carrying a radially-extending knife blade (32).

6. A method according to Claim 4 or 5, **characterised in that** the tube has an interlayer (21') between the inner and outer layers (11 and 12) to prevent bonding between the inner and outer layers.

7. A method according to Claim 6, **characterised in that** the interlayer (21') is of polyethylene.

8. A method according to any one of the preceding claims, **characterised in that** the outer layer (12) is end formed after removal of the part of the inner layer to close the one end (2) of the tube (1), and that a side opening (3) is formed in the tube towards the one end (2).

9. A method according to any one of the preceding claims, **characterised in that** the inner layer (11) is of a stiffer material that the outer layer (12).

## Patentansprüche

1. Verfahren zur Herstellung eines medizinisch-chirurgischen Tubus (1), beinhaltend den Schritt der Herstellung eines röhrenförmigen Teils mit einer inneren Schicht (11) und einer äußeren Schicht (12), **gekennzeichnet durch** das anschließende Entfernen mindestens eines Teils der inneren Schicht über einen Bereich (13) an einem Ende (2) des Tubus zum Zwecke der Verminderung der Steifigkeit des Tubus über diesen Bereich (13).

2. Verfahren nach Anspruch 1, **dadurch gekennzeichnet, dass** der Teil der inneren Schicht entfernt wird durch maschinelles Abtragen des Teils der inneren Schicht (11) über den Bereich (13).

3. Verfahren nach Anspruch 2, **dadurch gekennzeichnet, dass** der Teil der inneren Schicht (11) maschinell abgetragen wird unter Verwendung einer Maschine (20) mit einem rotierenden Fräskopf (21) mit einer Fräsoberfläche (22) an einer Endfläche.

4. Verfahren nach Anspruch 1, **dadurch gekennzeichnet, dass** der Teil der inneren Schicht (11) entfernt wird durch Abschneiden des Teils (23')von der verbleibenden inneren Schicht und dessen Herausziehen aus dem Tubus.

5. Verfahren nach Anspruch 4, **dadurch gekennzeichnet, dass** der Teil (23') der inneren Schicht (11) abgeschnitten wird unter Verwendung einer Maschine (30) mit einer rotierenden Spindel (31) welche eine radial hervorstehende Messerklinge (32) trägt.

6. Verfahren nach einem der Ansprüche 4 oder 5, **dadurch gekennzeichnet, dass** der Tubus eine Zwischenschicht (21') zwischen der inneren und äußeren Schicht (11 bzw. 12) aufweist zur Verhinderung des Verklebens der inneren mit der äußeren Schicht.

7. Verfahren nach Anspruch 6, **dadurch gekennzeichnet, dass** die Zwischenschicht (21') aus Polyäthylen besteht.

8. Verfahren nach einem der voranstehenden Ansprüche, **dadurch gekennzeichnet, dass** die äußere Schicht (12) endgeformt wird nach der Entfernung des Teils der inneren Schicht nahe des einen Endes (2) des Tubus (1), und dass eine seitliche Öffnung (3) im Tubus im Bereich des einen Endes (2) eingebracht wird.

9. Verfahren nach einem der voranstehenden Ansprüche, **dadurch gekennzeichnet, dass** die innere Schicht (11) aus einem steiferen Material als die äußere Schicht (12) besteht.

## Revendications

1. Procédé de fabrication d'un tube médico-chirurgical (1), le procédé comportant l'étape de formation d'un élément tubulaire avec une couche intérieure (11) et une couche extérieure (12), le procédé étant **caractérisé par** l'étape d'enlèvement ultérieur d'une partie au moins de la couche intérieure le long d'une zone (13) à une seule extrémité (2) dû tube de façon à réduire la rigidité du tube le long de la zone (13).

2. Procédé selon la revendication 1, **caractérisé en ce que** la partie de la couche intérieure est retirée par usinage de la partie de la couche intérieure (11) le long de la zone (13).

3. Procédé selon la revendication 2, **caractérisé en ce que** la partie de la couche intérieure (11) est usinée en utilisant une machine (20) comportant une tête de fraisage rotative (21) pourvue d'une surface de fraisage (22) sur une face d'extrémité.

4. Procédé selon la revendication 1, **caractérisé en ce que** la partie de la couche intérieure (11) est enlevée par coupage de la partie (23') du reste de la couche intérieure et en la tirant à l'extérieur du tube.

5. Procédé selon la revendication 4, **caractérisé en ce que** la partie (23') de la couche intérieure (11) est coupée en utilisant une machine (30) comportant un axe rotatif (31) portant une lame de couteau (32) s'étendant radialement.

6. Procédé selon la revendication 4 ou 5, **caractérisé en ce que** le tube comporte une couche intermédiaire (21') entre les couches intérieure et extérieure (11 et 12) pour empêcher une liaison entre les couches intérieure et extérieure.

7. Procédé selon la revendication 6, **caractérisé en ce que** la couche intermédiaire (21') est en polyéthylène.

8. Procédé selon l'une quelconque des revendications précédentes, **caractérisé en ce que** la couche extérieure (12) est formée à l'extrémité après l'enlèvement de la partie de la couche intérieure pour fermer l'extrémité (2) du tube (1), et **en ce qu'**une ouverture latérale (3) est ménagée dans le tube vers l'extrémité (2).

9. Procédé selon l'une quelconque des revendications précédentes, **caractérisé en ce que** la couche intérieure (11) est en un matériau plus rigide que la couche extérieure (12).
